# EUROPEAN PATENT SPECIFICATION

(11) **EP 2 683 351 B1**
(45) Date of publication and mention of the grant of the patent: **16.10.2019**
(21) Application number: 12704849.4
(22) Date of filing: 23.02.2012
(51) Int. Cl.: A61K 8/37, A61K 8/49, A61Q 17/04, A61K 8/35

(54) **USE OF A 2-METHYLSUCCINIC ACID DIESTER DERIVATIVE AS SOLVENT IN COSMETIC COMPOSITIONS; COSMETIC COMPOSITIONS CONTAINING THE SAME**
VERWENDUNG EINES DIESTER DERIVAT VON 2-METHYLSUCCINICSAÜRE ALS LÖSUNGSMITTEL IN KOSMETISCHEN ZUSAMMENSETZUNGEN; KOSMETISCHE ZUSAMMENSETZUNG ENZHALTEND DERGLEICHEN
UTILISATION D'UN DÉRIVÉ DE DIESTER DE L'ACIDE 2-MÉTHYLSUCCINIQUE COMME SOLVANT DANS DES COMPOSITIONS COSMÉTIQUES ET COMPOSITIONS COSMÉTIQUES LE CONTENANT

(30) Priority: 09.03.2011 FR 1151915; 11.03.2011 US 201161451850 P
(43) Date of publication of application: 15.01.2014
(73) Proprietor: L'Oréal, 75008 Paris (FR)
(72) Inventor: RICHARD, Hervé, F-93320 Gagny (FR); MULLER, Benoit, F-75019 Paris (FR)
(74) Representative: L'Oreal
(86) International application number: PCT/EP2012/053067
(87) International publication number: WO 2012/119861

(56) References cited:
- EP-A1- 0 860 164
- EP-A2- 0 212 729
- EP-A2- 0 801 943
- WO-A1-98/45244
- JP-A- 8 000 285
- JP-A- 10 287 620
- "Diethyl Succinate, Dimethyl Succinate" In: "International Cosmetics Ingredients Dictionary and Handbook", 2006, The cosmetic, toiletry and fragrance association, Washington D.C., XP002661784, vol. 1 the whole document

## Description

The present invention relates to the use of at least one 2-methylsuccinic acid diester derivative of formula (I), the definition of which will be given hereinbelow, in a composition comprising, in a cosmetically acceptable medium, at least one liquid fatty phase and at least one lipophilic active agent selected from lipophilic organic UV-screening agents, for dissolving the said active agent in the said liquid fatty phase and/or for improving the solubility of the said active agent in the liquid fatty phase.

The present invention relates especially to a composition comprising, in a cosmetically acceptable medium, at least one liquid fatty phase, characterized in that it contains at least one 2-methylsuccinic acid ester derivative of formula (I) and at least one lipophilic active agent selected from lipophilic organic UV-screening agents.

Many cosmetic or dermatological products are in various galenical forms comprising a liquid fatty phase, such as dispersions, oily solutions, oil/water emulsions, water/oil emulsions or multiple emulsions, or cream gels. Certain particularly advantageous cosmetic or dermatological active agents such as lipophilic organic screening agents are difficult to dissolve in the oily phase of these formulations and have a tendency on storage to form crystals or to precipitate especially in emulsions. Such phenomena are undesirable from the point of view of the stability of the formulation and/or as regards the consumer's comfort, insofar as they may destabilize the composition and/or affect the aesthetic appearance of the product and/or lead to cosmetic drawbacks on application to the skin and/or the hair or else limit the concentration of active agents in these formulations, which does not make it possible to obtain products that are sufficiently effective.

This is the case especially for care formulations containing lipophilic active agents such as active agents chosen from aminophenol derivatives, salicylic acid derivatives, N,N'-di(arylmethylene)ethylenediaminetriacetate derivatives, 2-amino-4-alkylaminopyrimidine 3-oxide derivatives, in particular 2-amino-4-dodecylaminopyrimidine 3-oxide, DHEA (dehydroepiandrosterone), derivatives thereof and chemical precursors thereof such as 7-hydroxy or 7-keto DHEA, or alternatively 3β-acetoxy-7-keto DHEA, cholesterol and derivatives thereof, plant sterols such as phytosterols and sitosterols and esters thereof, pentacyclic triterpenic acids, hydroxystilbenes, flavonoids, lipophilic organic UV-screening agents in antisun formulations, retinoids such as retinol and esters thereof or its precursors, carotenoids such as lycopene, and also fragrances, essential oils, hormones, vitamins and in particular vitamin E, and ceramides, or mixtures thereof.

In particular, the antisun compositions are often in the form of an emulsion of oil-in-water or water-in-oil type, gels or anhydrous products that contain, in varying concentrations, one or more organic and/or inorganic, insoluble and/or soluble, lipophilic and/or hydrophilic screening agents that are capable of selectively absorbing harmful UV radiation, these screening agents and the amounts thereof being selected as a function of the desired protection factor. Depending on their lipophilic or, on the contrary, hydrophilic nature, these screening agents may be distributed, respectively, either in the fatty phase or in the aqueous phase of the final composition.

Lipophilic screening agents are commonly used in sunscreen formulations. However, for a certain number of them, their photoprotective power in formulation is relatively limited in the usual cosmetic supports containing oils such as oxyethylenated or oxypropylenated fatty (mono/poly)alcohols (Cetiol HE from Henkel or Witconol APM from Witco) or alternatively fatty esters such as C12-C15 alkyl benzoates (Finsolv TN from Finetex), fatty acid triglycerides, for example Miglyol® 812 sold by the company Dynamit Nobel, or amino acid derivatives (Eldew SL205 from Ajinomoto) since the solubility of these screening agents in these oils commonly used in formulation is scarcely satisfactory. The consequence of this is either the appearance over time of crystallization in the formulations, which harms the quality, stability and efficacy of the antisun products, or limits the concentration of screening agents in the formulations, which does not make it possible to obtain products that are sufficiently effective.

Documents EP 0 801 943 and EP 0 860 164 disclose the use of diesters as a solvent for triazine-type UV-Filters in an oil phase. However, these solvents are not satisfactory enough.

There is thus a need to find novel solvents that can improve the solubility of lipophilic active agents and especially lipophilic screening agents in oils and in the supports of cosmetic or dermatological formulations containing them, without the drawbacks mentioned above.

The present invention relates to the use of at least one 2-methylsuccinic acid diester derivative of formula (I), the definition of which will be given hereinbelow, in a composition comprising, in a cosmetically acceptable medium, at least one liquid fatty phase and at least one lipophilic active agent, for dissolving the said active agent in the said liquid fatty phase and/or for improving the solubility of the said active agent in the liquid fatty phase.

The present invention relates especially to a composition comprising, in a cosmetically acceptable medium, at least one liquid fatty phase, characterized in that it contains at least one 2-methylsuccinic acid ester derivative of formula (I) and at least one lipophilic active agent selected from lipophilic organic UV-screening agents.

The present invention also relates to the use of at least one derivative of formula (I) in a composition comprising, in a cosmetically acceptable medium, at least one liquid fatty phase and at least one lipophilic active agent selected from lipophilic organic UV-screening agents, to improve the efficacy of the said active agent and/or the cosmetic qualities and/or the stability of the said composition.

The present invention also relates to the use of at least one derivative of formula (I) in a composition comprising, in a cosmetically acceptable medium, at least one liquid fatty phase and at least one lipophilic organic UV-screening agent, to improve the sun protection factor.

Other characteristics, aspects and advantages of the invention will emerge on reading the detailed description that follows.

The term "cosmetically acceptable" means compatible with the skin and/or its integuments, having a pleasant colour, odour and feel and not causing any unacceptable discomfort (stinging, tautness or redness) liable to dissuade the consumer from using this composition.

For the purposes of the present patent application, the term "liquid fatty phase" means a fatty phase that is liquid at room temperature (25°C) and atmospheric pressure (760 mmHg), composed of one or more mutually compatible fatty substances that are liquid at room temperature, also known as oils.

The term "lipophilic active agent" means any cosmetic or dermatological organic active agent which can be fully dissolved in the molecular state in a liquid fatty phase or which can be dissolved in colloidal form (for example in micellar form) in a liquid fatty phase.

The term "dissolving a lipophilic active agent in the liquid fatty phase" means fully dissolving the said active agent in the molecular state in the liquid fatty phase, or else dissolving it in colloidal form (for example in micellar form) in the liquid fatty phase.

According to one particular variant, the term "lipophilic active agent" means any cosmetic or dermatological organic active agent that has solubility in water of less than 0.5% by weight and solubility of less than 10% by weight in the majority of organic oils such as liquid paraffin, fatty alkyl benzoates and fatty acid triglycerides, for example Miglyol® 812 sold by the company Dynamit Nobel. This solubility, determined at 70°C, is defined as the amount of product in solution in the solvent at equilibrium with an excess of solid in suspension after returning to room temperature. It may be readily evaluated in the laboratory.

The 2-methylsuccinic acid diester derivatives in accordance with the invention are chosen from those corresponding to the general formula (I) below, and also optical isomers thereof and/or solvates thereof: 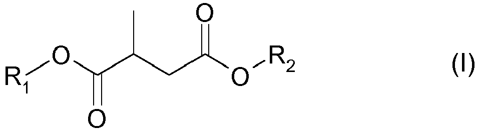 in which:
R₁ and R₂, which may be identical or different, denote a linear or branched C₁-C₁₂ alkyl radical,
R₁ and R₂ not being able simultaneously to denote a methyl radical.

In the formula (I), mention may be made especially, among the alkyl groups, of methyl, ethyl, n-propyl, isopropyl, n-butyl, isobutyl, tert-butyl, 2-butyl, n-pentyl, isopentyl, n-hexyl, n-heptyl, n-octyl, 2-ethylhexyl, decyl, dodecyl groups.

In the context of the invention, the term "2-methylsuccinic acid diester derivative of formula (I)" means any mesomeric, tautomeric or optical isomer form.

Among the compounds of formula (I), compounds (a) to (ab) below will more particularly be used:

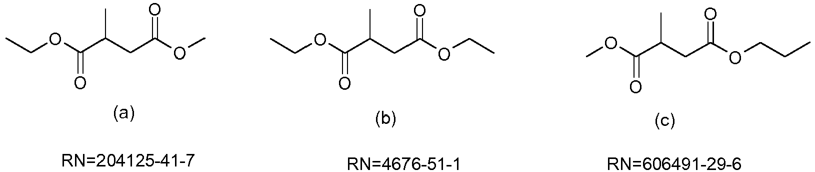

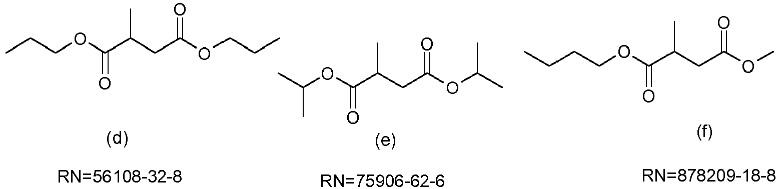

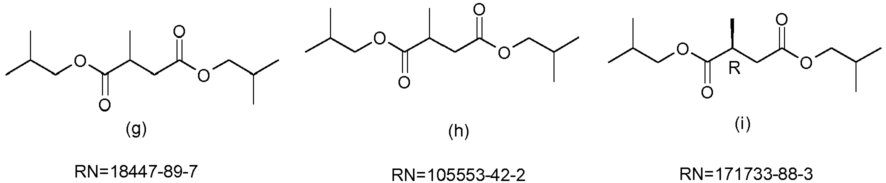

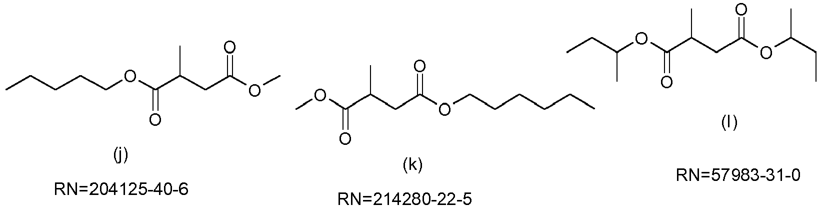

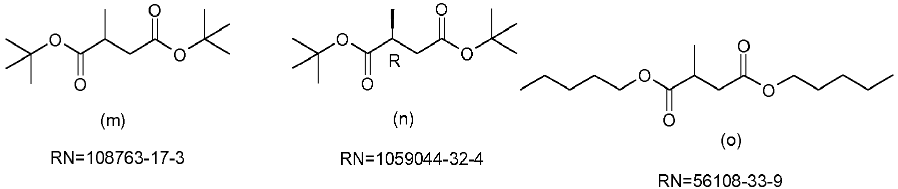

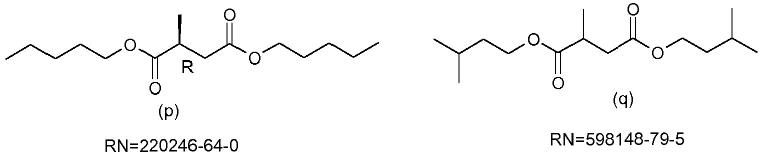

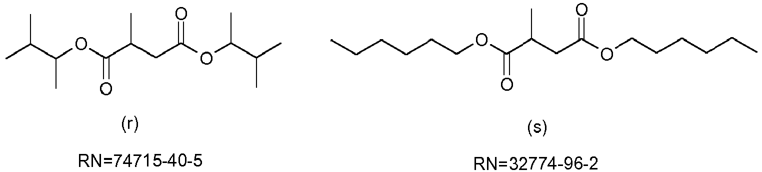

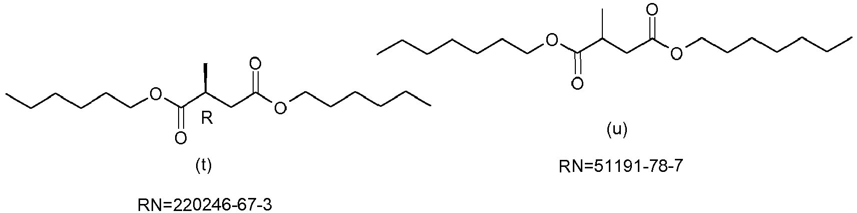

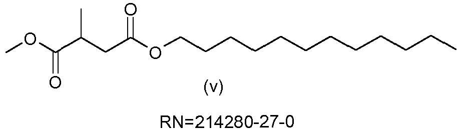

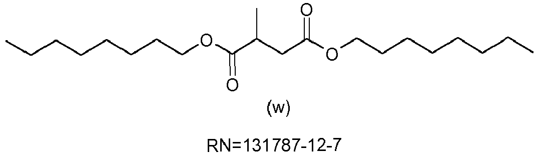

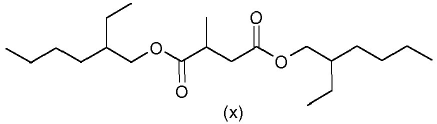

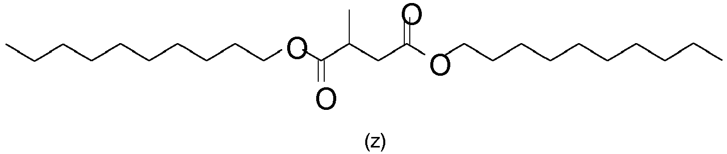

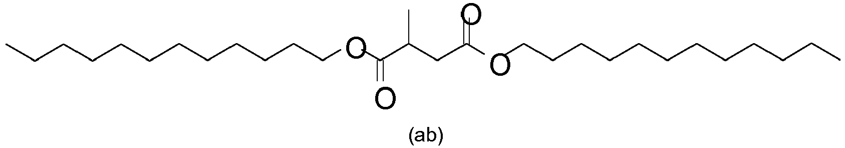

According to one particularly preferred variant of the invention, compounds (g), (j), (k), (I), (o), (q), (s), (u), (v), (w), (x), (z) and (ab) will be used more particularly, and even more particularly compounds (s), (w) and (x).

The "solvates" represent hydrates and also the combination with solvents, in particular with linear or branched C₁-C₄ alcohols such as ethanol, isopropanol and n-propanol.

The derivatives of formula (I) in accordance with the invention are preferably present in the compositions of the invention in contents ranging from 1% to 30% by weight and more preferentially from 3% to 20% by weight, relative to the total weight of the composition.

The derivatives of formula (I), the syntheses of which are described in the following articles: Chemistry-A European J., 14 (10), page 3118 (2008), J. Organometallic Chem., 195 (3), pages 337-46 (1980); J. Molecular Catalysis A: Chem., 137 (1-3), pages 263-7 (1999), Acta Chemica Scandinavia, 13, pages 1943-54 (1959); and in patents US 4730080 (2005) and JP 08217770, may be readily obtained:
- either by the catalytic reduction of a diester of itaconic acid of formula (II), with or without solvent, at a temperature of between 20°C and 150°C, according to the following scheme: 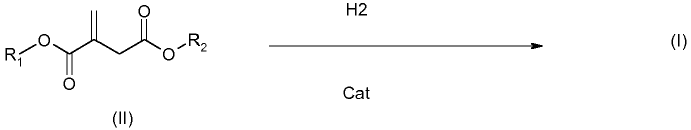
- or (when R₁ = R₂) from 2-methylsuccinic acid of formula (III) by diesterification with the alcohol of formula (IV) in the presence of a catalyst according to the following scheme:

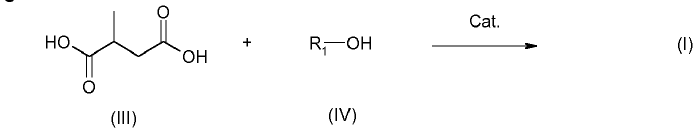

This same 2-methylsuccinic acid may be obtained by catalytic hydrogenation of itaconic acid according to the article Advanced Materials Research, 20-21 (Biohydrometallurgy), pages 603-6 (2007).

Compounds (x), (z) and (ab) are novel and constitute another subject of the invention.

According to one particular form of the invention, the derivative(s) of formula (I) in accordance with the invention are present, alone or as a mixture, in an amount that is sufficient to dissolve by themselves (without it being necessary to use another solvent) the total amount of lipophilic active agent(s) present in the composition.

According to another particular form of the invention, the derivative(s) of formula (I) in accordance with the invention constitute the sole solvent for the lipophilic active agent(s).

In this case, the liquid fatty phase may be formed by the derivative(s) of formula (I) and the lipophilic active agent(s) dissolved in the said phase.

Among the lipophilic active agents that may be used in accordance with the invention, use will be made of lipophilic organic UV-screening agents. They may be chosen from para-aminobenzoic acid derivatives, salicylic derivatives, cinnamic derivatives, benzophenone and aminobenzophenone derivatives, anthranilic derivatives, dibenzoylmethane derivatives, β,β-diphenylacrylate derivatives, benzylidenecamphor derivatives, benzotriazole derivatives, triazine derivatives, bis-resorcinyl triazine derivatives, imidazoline derivatives, benzalmalonate derivatives, 4,4-diarylbutadiene derivatives, benzoxazole derivatives, diphenyl butadiene malonate or malononitrile derivatives, chalcone derivatives and merocyanin derivatives, and mixtures thereof.

Among the lipophilic organic UVA screening agents that are capable of absorbing UV from 320 to 400 nm, mention may be made of:

### Dibenzoylmethane derivatives:

- 4-isopropyldibenzoylmethane, sold under the name Eusolex 8020 by the company Merck, and corresponding to the following formula: 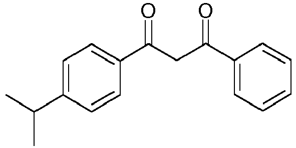
- 1-(4-methoxy-1-benzofuran-5-yl)-3-phenylpropane-1,3-dione, provided for sale by the company Quest under the name of Pongamol, of formula: 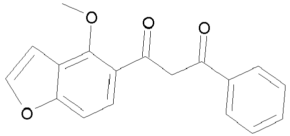
- 1-(4-tert-butylphenyl)-3-(2-hydroxyphenyl)propane-1,3-dione of formula: 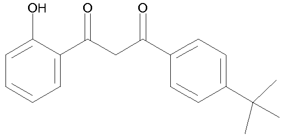
- butylmethoxydibenzoylmethane or avobenzone sold especially under the trade name Parsol 1789 by DSM,

### Aminobenzophenones:

- n-hexyl 2-(4-diethylamino-2-hydroxybenzoyl)benzoate sold under the trade name Uvinul A+ by BASF;
- 1,1'-(1,4-piperazinediyl)bis[1-[2-[4-(diethylamino)-2-hydroxybenzoyl]phenyl] methanone (CAS 919803-06-8) in its micronized or non-micronized form.

### Anthranilic derivatives:

menthyl anthranilate sold under the trade name Neo Heliopan MA by Symrise,

### 4.4-Diarylbutadiene derivatives:

1,1-dicarboxy(2,2'-dimethylpropyl)-4,4-diphenylbutadiene,

### Merocyanin derivatives:

- octyl 5-N,N-diethylamino-2-phenylsulfonyl-2,4-pentadienoate,
- the lipophilic merocyanin derivatives of general formula (V): 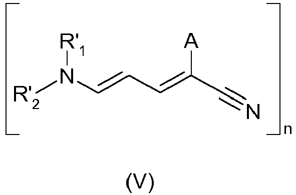
in which:
- A is the -C=N or -(C=O)OR'₃ group;
- R'₁ and R'₂, which may be identical or different, represent H, a linear or branched C₁-C₂₂ alkyl radical or a C₃-C₈ cycloalkyl radical optionally substituted with C₁-C₄ alkyl radicals;
- R'₁ and R'₂ may form, together with the nitrogen, a ring containing the group - (CH₂)ₘ- which may be interrupted with one or more -O- or -S- or with -NH-;
- the radical R'₁ may form, with the carbon alpha to the nitrogen, a ring containing the group -(CH₂)ₘ- which may or may not be interrupted with one or more -O- or -S- or with -NH-;
- R'₃ represents a linear or branched C₁-C₂₂ alkyl radical or a C₃-C₈ cycloalkyl radical optionally substituted with C₁-C₄ alkyl radicals;
- n is 1 or 2;
- m is an integer ranging from 3 to 7;
with the proviso that:
(i) when n = 2, R'₁, R'₂ or R'₃ is an alkyl diradical or R'₁ and R'₂, together with two nitrogen atoms, form a cyclic divalent radical -(CH₂)ₘ-;
(ii) R'₁ and R'₂ are not simultaneously a hydrogen atom;

The compounds of formula (V) may be in the E,E-, E,Z- or Z,Z- geometrical isomeric forms.

The compounds of formula (V) that are particularly preferred are those for which the following conditions are satisfied:
- R'₁ and R'₂ denote a linear or branched C₁-C₃ alkyl or a C₅-C₆ cycloalkyl radical,
   R'₃ denotes a linear or branched C₁-C₈ alkyl radical,
- n is equal to 1 or 2.

The compounds of formula (V) that are more particularly preferred are those of formulae (Ma) to (Mdd) below, or the E,E-, E,Z- or Z,Z- isomers thereof:

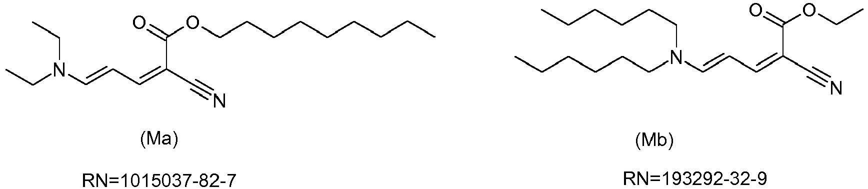

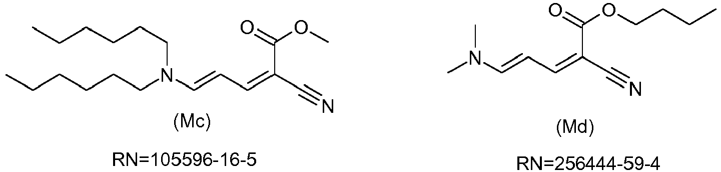

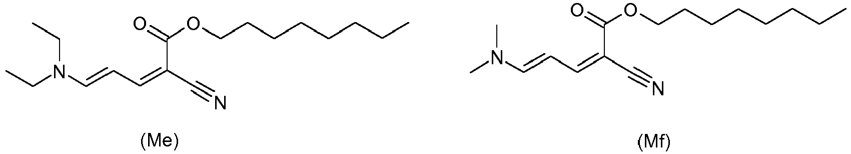

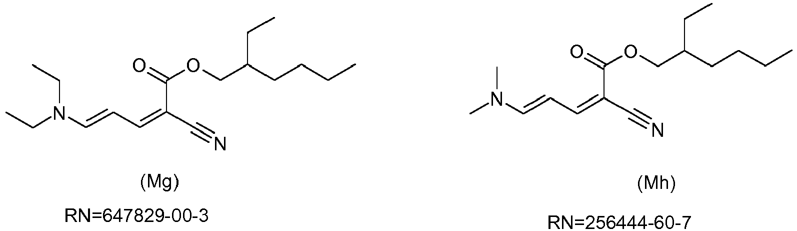

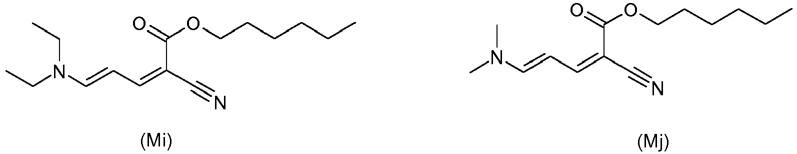

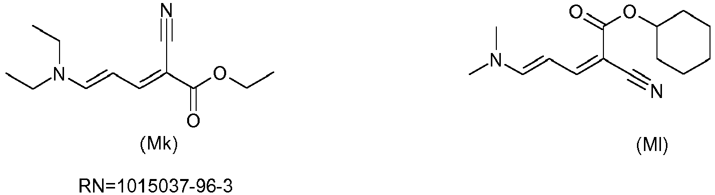

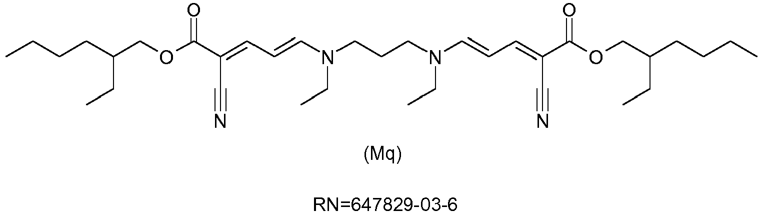

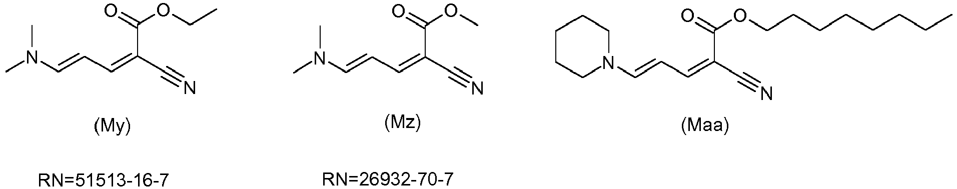

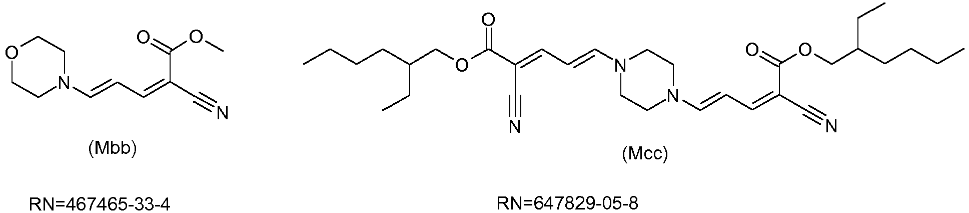

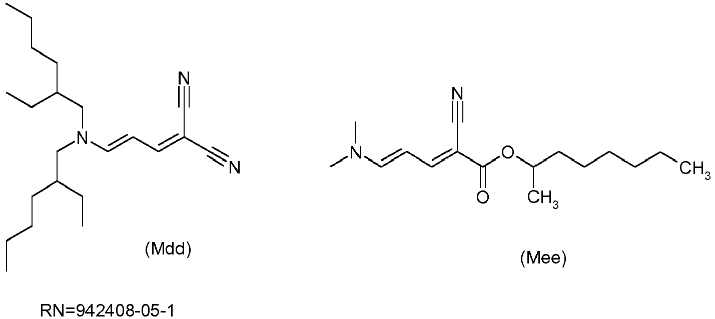

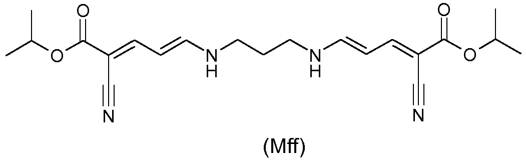

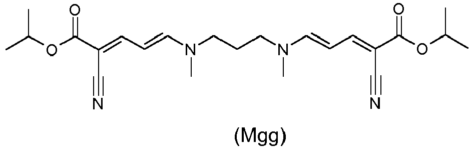

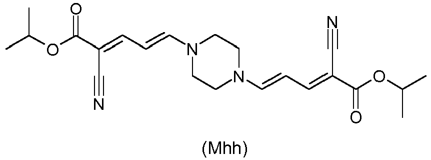

The following ten compounds, or the E,E-, E,Z- or Z,Z- isomers thereof, will be even more particularly preferred:

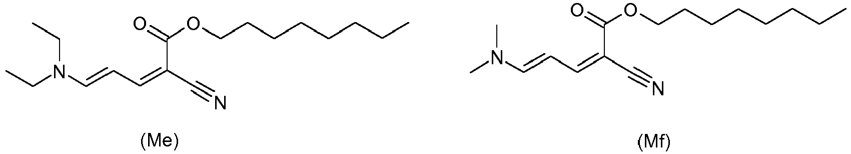

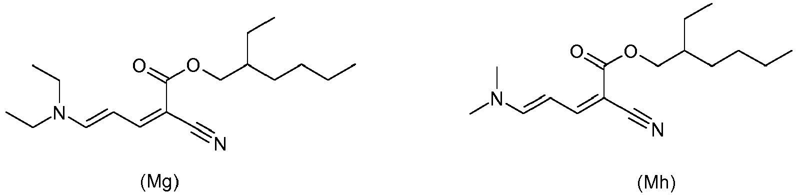

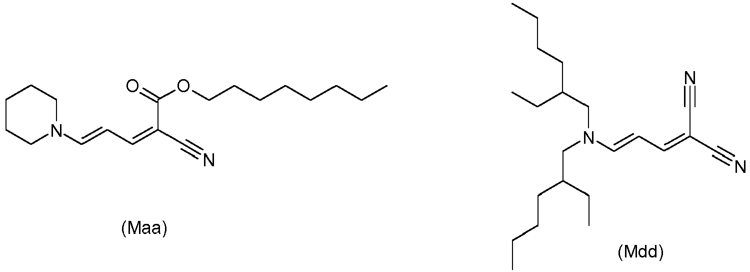

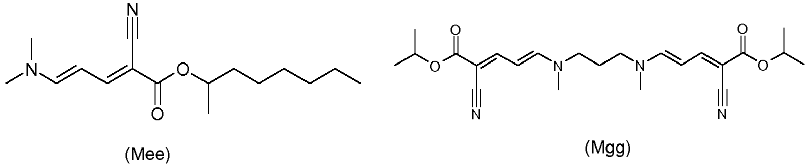

The merocyanin derivatives of formula (V) can be prepared according to a method described in patents US 4 045 229 and US 4 195 999, according to the following scheme (Route 1): 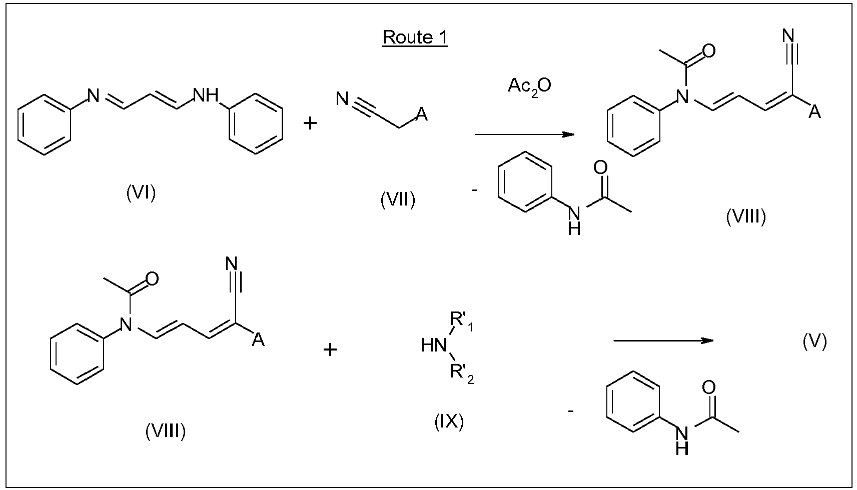 in which the radicals R'₁, R'₂ and A have the same meaning as in formula (V).

The derivatives of formula (V) can be prepared according to a method described in patent WO 00/20388, according to the following scheme (Route 2): 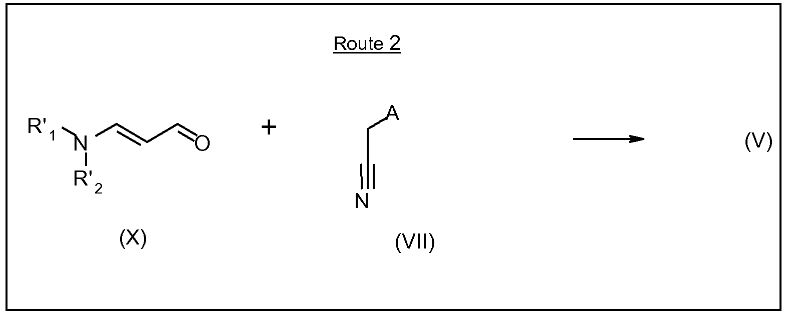 in which the radicals R'₁, R'₂ and A have the same meanings as in formula (V).

The derivatives of formula (V), in which A represents -(C=O)OR'₃ and R'₃ denotes an alkyl containing at least 3 carbon atoms, can be obtained by transesterification according to the scheme below, which route is described in patent US 2008076940 and can use, inter alia, as catalyst, titanium(IV) isopropoxide (Route 3):

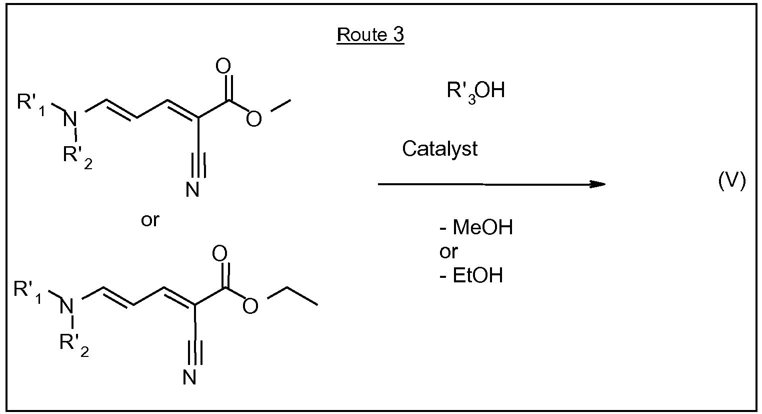

The derivatives of formula (V) in which R'₁ and R'₂ denote an alkyl containing at least 3 carbon atoms can be obtained by transamination, according to the scheme below (Route 4):

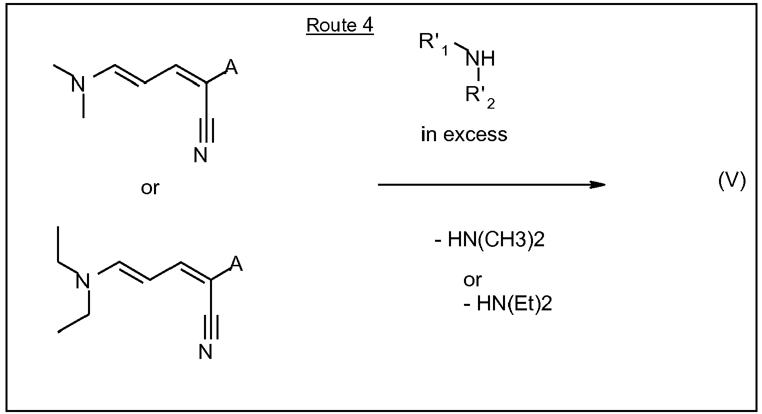

Among the lipophilic UVB-screening agents that are capable of absorbing UV from 280 to 320 nm,
mention may be made of:
para-aminobenzoic acid derivatives (or para-aminobenzoates):
Ethyl PABA,
Ethyl dihydroxypropyl PABA,
Ethylhexyl Dimethyl PABA (Escalol 507 from ISP),

### Salicylic derivatives:

- Homosalate sold under the name Eusolex HMS by Rona/EM Industries,
- Ethylhexyl salicylate sold under the name Neo Heliopan OS by Symrise,
- Dipropylene glycol salicylate sold under the name Dipsal by Scher, TEA salicylate sold under the name Neo Heliopan TS by Symrise.

### Cinnamic derivatives:

Ethylhexyl methoxycinnamate sold in particular under the trade name Parsol MCX by DSM,
Isopropyl methoxycinnamate,
Isoamyl methoxycinnamate sold under the trade name Neo Heliopan E 1000 by Symrise,
Diisopropyl methylcinnamate,
Cinoxate,
Glyceryl ethylhexanoate dimethoxycinnamate,
(2E)-3-(2,4,5-trimethoxyphenyl)prop-2-enoic acid of the following formula:

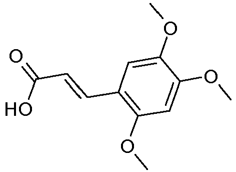

### ββ-Diphenylacrylate derivatives:

Octocrylene sold especially under the trade name Uvinul N539 by BASF,
Etocrylene sold especially under the trade name Uvinul N35 by BASF,

### Benzylidenecamphor derivatives:

3-Benzylidenecamphor manufactured under the name Mexoryl SD by Chimex,
Methylbenzylidenecamphor sold under the name Eusolex 6300 by Merck,
Polyacrylamidomethylbenzylidenecamphor manufactured under the name Mexoryl SW by Chimex,

### Triazine derivatives:

- ethylhexyltriazone sold in particular under the trade name Uvinul T150 by BASF,
   diethylhexylbutamidotriazone sold under the trade name Uvasorb HEB by Sigma 3V,
   2,4,6-tris(dineopentyl 4'-aminobenzalmalonate)-s-triazine,
   2,4,6-tris(diisobutyl 4'-aminobenzalmalonate)-s-triazine,
   2,4-bis(dineopentyl 4'-aminobenzalmalonate)-6-(n-butyl 4'-aminobenzoate)-s-triazine,
   bis(butyl benzoate)diaminotriazine aminopropyltrisiloxane,

### Imidazoline derivatives:

Ethylhexyldimethoxybenzylidenedioxoimidazoline propionate,

### Benzalmalonate derivatives:

Polyorganosiloxanes containing benzalmalonate functions, such as Polysilicone-15, sold under the trade name Parsol SLX by DSM Dineopentyl 4'-methoxybenzalmalonate,

Among the lipophilic organic UV-screening agents, use will be made of broad-spectrum lipophilic screening agents. Among the broad-spectrum lipophilic organic UV-screening agents capable of absorbing UVA and UVB, mention may be made of:

### Benzophenone derivatives:

Benzophenone-1 sold under the trade name Uvinul 400 by BASF,
Benzophenone-2 sold under the trade name Uvinul D50 by BASF,
Benzophenone-3 or oxybenzone sold under the trade name Uvinul M40 by BASF,
Benzophenone-6 sold under the trade name Helisorb 11 by Norquay,
Benzophenone-8 sold under the trade name Spectra-Sorb UV-24 by American Cyanamid,
Benzophenone-10,
Benzophenone-11,
Benzophenone-12,

### Benzotriazole derivatives:

Drometrizole trisiloxane sold under the name Silatrizole by Rhodia Chimie,
Bumetrizole sold under the name Tinoguard AS by BASF,

### Bis-resorcinyl triazine derivatives:

Bis(ethylhexyloxyphenol)methoxyphenyltriazine sold under the trade name Tinosorb S by BASF,

### Benzoxazole derivatives:

2,4-bis[5-(1-dimethylpropyl)benzoxazol-2-yl(4-phenyl)imino]-6-(2-ethylhexyl)imino-1,3,5-triazine sold under the name Uvasorb K2A by Sigma 3V.

### Diphenylbutadiene derivatives:

The derivatives of the diphenylbutadiene malonate or malononitrile family are the derivatives of general formula (XI) below: 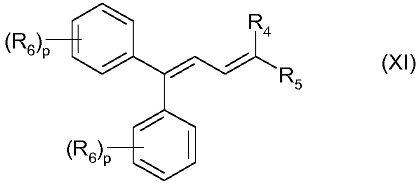
in which R₆ represents a C₁-C₂ alkyl group or a C₁-C₂ alkoxy group and p is equal to 0, 1 or 2;
R₄ and R₅, which may be identical or different, represent -COOR₇, -(C=O)NHR₇, -(C=O)R₇ or -CN, in which R₇ represents a linear or branched alkyl group containing from 1 to 12 carbon atoms which may contain silane, siloxane or polysiloxane groups.

Among the diphenylbutadiene malonate or malononitrile derivatives that may especially be mentioned, in a non-limiting manner, are:
- dimethyl 2-(3,3-diphenylprop-2-enylidene)malonate
- diisobutyl 2-(3,3-diphenylprop-2-enylidene)malonate
- bis(1,3-dimethylbutyl) 2-(3,3-diphenylprop-2-enylidene)malonate
- dineopentyl 2-(3,3-diphenylprop-2-enylidene)malonate
- methyl (2Z)-2-cyano-5,5-diphenylpenta-2,4-dienoate
- ethyl (trimethylsilyl)methyl (2Z)-2-(3,3-diphenylprop-2-enylidene)malonate
- (2E)-2-cyano-5,5-diphenyl-N-(3-{1,3,3,3-tetramethyl-1-[(trimethylsilyl)oxy]disiloxanyl}propyl)penta-2,4-dienamide
- ethyl 2-methyl-3-{1,3,3,3-tetramethyl-1-[(trimethylsilyl)oxy]disiloxanyl}propyl (2E)-2-(3,3-diphenylprop-2-enylidene)malonate
- ethyl (2Z)-5,5-diphenyl-2-{[(3-{1,3,3,3-tetramethyl-1-[(trimethylsilyl)oxy]disiloxanyl}propyl)amino]carbonyl}penta-2,4-dienoate

Among the diphenylbutadiene derivatives mentioned above, use will be made in particular of dineopentyl 2-(3,3-diphenylprop-2-enylidene)malonate corresponding to formula (Xla) below:

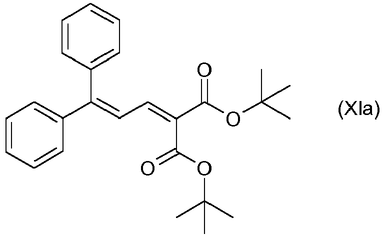

It is known practice to use these diphenylbutadiene derivatives in antisun compositions: patent EP 0 916 335 describes the carbon-based derivatives and the modes for obtaining the same, and patents EP 1 535 947 and EP 1 535 925 describe the siloxane and silane derivatives, respectively.

### Chalcone derivatives:

The derivatives of the chalcone family are the derivatives of general formula (XII) below: 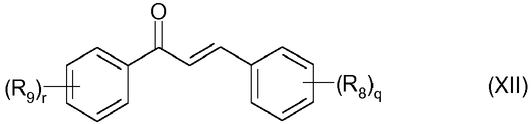
in which the radicals R₈ and R₉ denote, independently of each other, a hydroxyl radical, a linear or branched C₁-C₁₂ alkyl or alkenyl group, a linear or branched C₁-C₁₂ alkoxy group or a linear or branched C₂-C₂₀ acyloxy group;
q and r = 0, 1, 2, 3 or 4;
it being understood that q and/or r is other than 0.

Among the chalcone derivatives that may especially be mentioned, in a non-limiting manner, are:
- 2'-hydroxychalcone
- 4'-hydroxychalcone
- 4'-methoxychalcone
- 2'-hydroxy-4-methoxychalcone
- 2'-hydroxy-4-hexyloxychalcone
- 2'-hydroxy-4-methylchalcone
- 2'-hydroxy-3-hexyloxychalcone
- 2'-hydroxy-4'-hexyloxy-4-methylchalcone
- 2'-hydroxy-4'-hexanoyloxy-4-methoxychalcone
2',4',4-trihydroxy-3,3'-diallylchalcone (known under the name Kazonol)
2',4',4-trihydroxy-5'-(3-methyl-2-butene)chalcone (known under the name Broussochalcone B)
2',3',4',6',4-pentahydroxychalcone (known under the name Carthamin).

Among the chalcone derivatives mentioned above, use will be made in particular of 4'-hydroxychalcone corresponding to formula (Xlla) below: 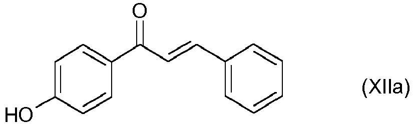 or alternatively 2',3',4',6',4-pentahydroxychalcone (known under the name Carthamin) corresponding to formula (Xllb) below:

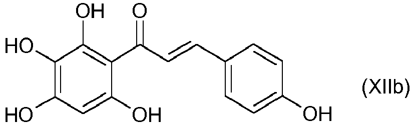

It is known practice to use these chalcone derivatives in antisun compositions, especially in patents FR 2 555 167, FR 2 602 228 and FR 2 608 150.

The lipophilic organic UV-screening agents are generally present in the compositions according to the invention in proportions ranging from 0.01% to 20% by weight relative to the total weight of the composition, and preferably ranging from 0.1% to 10% by weight relative to the total weight of the composition.

The lipophilic organic UV-screening agents that may be used in the context of the invention are chosen in particular from dibenzoylmethane derivatives, triazine derivatives, bis-resorcinyl triazine derivatives, benzotriazole derivatives, chalcone derivatives, benzophenone or aminobenzophenone derivatives, and derivatives of the diphenylbutadiene malonate or malononitrile family, or mixtures thereof.

Preferably, the lipophilic organic UV-screening agents that may be used in the context of the invention are chosen in particular from triazine derivatives and bis-resorcinyl triazine derivatives.

The composition according to the invention may also comprises additional lipophilic active agents which may be chosen from aminophenol derivatives, salicylic acid derivatives, N,N'-di(arylmethylene)ethylenediaminetriacetate derivatives, 2-amino-4-alkylaminopyrimidine 3-oxide derivatives, flavonoids, retinoides such as retinol and its esters or its precursors , carotenoids such as lycopene, and also fragrances, essential oils, hormones, vitamins, in particular vitamin E, and ceramides, or mixtures thereof.

The aminophenol derivatives used are more particularly the derivatives of formula (XIII) below: 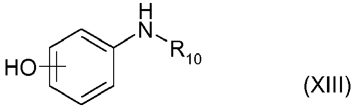 in which R₁₀ is a radical chosen from the group formed by the radicals (Aa), (Ab) and (Ac) below:
(Aa) -CO-NR₁₁R₁₂
(Ab) -CO-O-R₁₃
(Ac) -SO₂R₁₃

in which R₁₁ represents a hydrogen atom or a linear or branched, saturated or unsaturated, optionally hydroxylated C₁-C₆ alkyl radical,
R₁₂ represents a hydrogen atom or a radical chosen from saturated or unsaturated, linear, cyclic or branched, optionally hydroxylated C₁-C₃₀ alkyl radicals, and
R₁₃ represents a radical chosen from saturated or unsaturated, linear, cyclic or branched, including fused polycyclic, optionally hydroxylated C₁-C₃₀ alkyl radicals.

In formula (XIII), among the linear or branched alkyl radicals R₁₂ or R₁₃ containing from 1 to 30 carbon atoms, mention may be made advantageously of methyl, ethyl, propyl, isopropyl, butyl, tert-butyl, hexyl, octyl, nonyl, 2-ethylhexyl, dodecyl, hexadecyl, behenyl, octadecyl and 2-butyloctyl radicals. Preferably, these radicals contain from 1 to 12 carbon atoms. Even more preferentially, the alkyl radical generally comprises from 1 to 6 carbon atoms. Lower alkyl radicals that may be mentioned include methyl, ethyl, propyl, isopropyl, tert-butyl and hexyl radicals.

When R₁₂ or R₁₃ is unsaturated, a radical containing one or more ethylenic unsaturations is preferred, more particularly such as the allyl radical.

When R₁₂ or R₁₃ is cyclic, mention may be made especially of the cyclohexyl, cholesteryl or tert-butylcyclohexyl radical.

When R₁₂ or R₁₃ is hydroxylated, it preferably comprises from 1 to 6 carbon atoms and from 1 to 5 hydroxyl groups. Among the monohydroxyalkyl radicals, preference is given to a radical preferably containing 1 or 3 carbon atoms, especially hydroxymethyl, 2-hydroxyethyl or 2- or 3-hydroxypropyl radicals.

Among the polyhydroxyalkyl radicals, preference is given to a radical containing from 3 to 6 carbon atoms and from 2 to 5 hydroxyl groups, such as the 2,3-dihydroxypropyl, 2,3,4-trihydroxybutyl, 2,3,4,5-tetrahydroxypentyl and 2,3,4,5,6-pentahydroxyhexyl radicals.

Alkoxy radicals are alkyl radicals, especially as described above, preceded by an oxygen atom.

Preferably, the aminophenol derivatives used in the present patent application are those for which at least one and preferably all the conditions below are satisfied:
- the -OH function on the phenyl radical is in the ortho position or, advantageously, in the para position,
- R₁₀ is chosen from the radicals (Aa) and (Ab).

Among the linear or branched alkyl radicals R₁₁, mention may be made of methyl, ethyl, propyl, isopropyl, tert-butyl and hexyl radicals.

The aminophenol derivative preferably used in the said composition is a para-aminophenol derivative, and even more preferably is N-ethoxycarbonyl-4-para-aminophenol of formula (XIIIa): 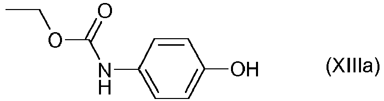 or N-cholesteryloxycarbonyl-4-para-aminophenol of formula (XIIIb):

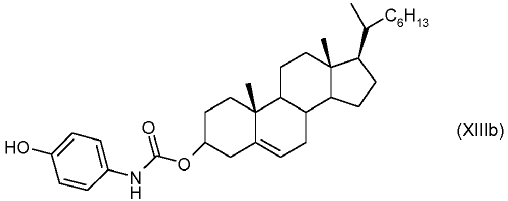

These aminophenol derivatives, and the process for preparing them, are described in patent applications WO 99/10318 and WO 99/32077.

These derivatives have a relatively long hydrocarbon-based chain, preferably an alkoxycarbonyl chain, linked to the nitrogen atom. They have the drawback of being sparingly soluble or even totally insoluble either in water or in the fatty phase of the type used in the present patent application. Their introduction into cosmetic compositions requires, for compounds with a short hydrocarbon-based chain, dissolution in aqueous-alcoholic solution, which is not always desirable when the composition is intended, for example, to be applied to the contour of the eyes.

For their part, long hydrocarbon-chain compounds are insoluble in oils on account of their steric bulk, and have a tendency to recrystallize water.

The compositions according to the present invention comprising such an aminophenol derivative may be used as depigmenting or bleaching agent in a cosmetic and/or dermatological composition.

The salicylic acid derivatives are the derivatives of formula (XIV) below: 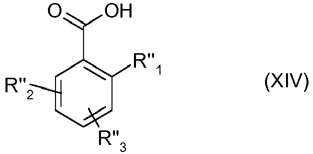 in which:
R"₁ represents a hydroxyl radical or an ester of formula -O-CO-R"₄ in which R"₄ is
   - a saturated or unsaturated aliphatic radical comprising from 1 to 26 carbon atoms and preferably from 1 to 18 carbon atoms,
   - an amine or thiol function optionally substituted with an alkyl radical comprising from 1 to 18 carbon atoms and preferably from 1 to 12 carbon atoms,
R"₂ and R"₃, independently of one another, are in position 3, 4, 5 or 6 on the benzene ring and represent, independently of one another, a hydrogen atom or a radical: -(O)ₛ-(CO)ₜ-R"₅
in which s and t, independently of each other, are each an integer equal to 0 or 1,
and R"₅ represents:
   - a hydrogen,
   - a linear, branched or cyclic saturated aliphatic radical comprising from 1 to 18 carbon atoms,
   - an unsaturated radical comprising from 3 to 18 carbon atoms, bearing one to nine conjugated or unconjugated double bonds,
   these radicals possibly being substituted with at least one substituent chosen from halogen atoms (fluorine, chlorine, bromine or iodine), trifluoromethyl or hydroxyl radicals in free form or esterified with an acid comprising from 1 to 6 carbon atoms, or carboxyl in free form or esterified with a lower alcohol comprising from 1 to 6 carbon atoms, or an aromatic radical comprising from 6 to 10 carbon atoms,
R"₂ and R"₃ cannot simultaneously represent a hydrogen atom.

Preferably, the salicylic acid derivative is such that R"₅ represents a saturated aliphatic radical comprising from 3 to 15 carbon atoms.

Preferably, the salicylic acid derivative is such that R"₁ represents a hydroxyl radical.

According to one preferred embodiment of the invention, the derivatives are 5-n-octanoylsalicylic acid, 5-n-decanoylsalicylic acid, 5-n-dodecanoylsalicylic acid, 5-n-octylsalicylic acid, 5-n-heptyloxysalicylic acid, 4-n-heptyloxysalicylic acid, 5-tert-octylsalicylic acid, 3-tert-butyl-5-methylsalicylic acid, 3-tert-butyl-6-methylsalicylic acid, 3,5-diisopropylsalicylic acid, 5-butoxysalicylic acid, 5-octyloxysalicylic acid, 5-propanoylsalicylic acid, 5-n-hexadecanoylsalicylic acid, 5-n-oleoylsalicylic acid, 5 benzoylsalicylic acid, their monovalent and divalent salts, and mixtures thereof.

It is most particularly preferred to use 2-hydroxy-5-octanoylbenzoic acid, which is sold under the trade name Mexoryl SAB by the company Chimex. It corresponds to formula (XIVa) below:

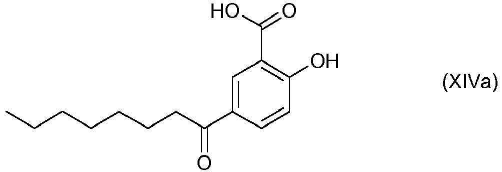

It is known practice to use salicylic acid derivatives in topical compositions, for example as keratolytic agents for treating acne or as anti-ageing agents: patent applications FR-A-2 581 542 and EP-A-378 936 describe such derivatives.

Salicylic acid derivatives are of great interest especially for preventing or repairing the main signs of ageing of the skin, namely wrinkles and fine lines, disorganization of the "grain" of the skin, modification of the complexion of the skin and loss of firmness and tonicity of the skin. However, the use of these derivatives poses a problem insofar as, when they are introduced into such topical compositions, they do not dissolve, but remain in the form of crystals, which makes the use of the composition containing them inefficient for treating the skin.

In general, these derivatives are dissolved in lower alcohols such as ethanol or isopropanol or in solvents such as octyldodecanol, certain glycols or short-chain (less than C₁₂) fatty alcohols. However, these lower alcohols have the drawback of drying out and irritating the skin: it is thus preferred to avoid using them in body care and/or facial care products. In addition, these solubilizers can only be introduced in small amounts, otherwise they run the risk of impairing the cosmetic qualities (drying-out of the skin) and the stability of the compositions containing them.

The concentration of salicylic acid derivatives in the composition according to the present invention is preferably from 0.001% to 15% by weight and even more preferentially from 0.1% to 5% by weight relative to the total weight of the composition.

The composition according to the invention comprising at least one salicylic derivative may be used as a cosmetic or dermatological composition, especially for caring for, protecting, cleansing and/or making up human keratin materials (skin, nails, lips and keratin fibres such as hair and eyelashes), and especially for combating the signs of ageing of the skin and/or for smoothing out facial and/or bodily skin and/or for treating skin wrinkles and fine lines and/or for stimulating the process of epidermal renewal and/or for depigmenting and bleaching the skin and/or for treating acne and/or for treating skin disorders.

These derivatives are described, along with a process for their preparation, in patent application WO 94/11338.

The derivatives of the 2-amino-4 alkylaminopyrimidine 3-oxide family are the derivatives of general formula (XV) below: 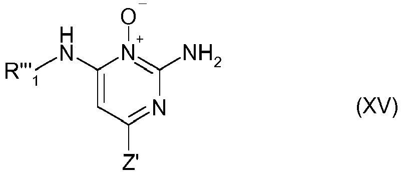 in which
R'"₁ represents
   - an alkyl radical comprising from 1 to 20 carbon atoms,
   - a C₁-C₂₀ alkanoyl radical,
   - or C(O)NHR'''₃ with R'"₃ representing a C₄-C₁₆ chain,
Z' represents a hydrogen atom or a radical -OR'"₂ in which R'"₂ represents an alkyl group containing from 1 to 12 carbon atoms,
and also the acylated forms thereof or the addition salts thereof with acids.

Preferably, R"'₂ is chosen from a hydrogen atom or from ethyl, propyl, butyl, pentyl and hexyl radicals.

Even more preferably, it is N-(2-amino-3-oxidopyrimidin-4-yl)-N'-dodecylurea of formula (XVa) below:

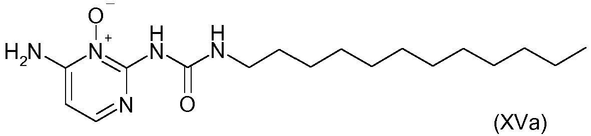

The derivatives of the 2-amino-4-alkylaminopyrimidine 3-oxide family may especially be used in or for the preparation of a cosmetic or dermatological composition in accordance with the present invention for preventing and treating problems associated with sensitive skin and skin disruptions such as cutaneous discomfort, taut skin, itchy skin, swollen skin, redness of the skin and the sensation of hot skin.

The concentration of derivatives of the 2-amino-4-alkylaminopyrimidine 3-oxide family in the composition according to the present invention is preferably from 0.001% to 15% by weight and even more preferentially from 0.1% to 5% by weight relative to the total weight of the composition.

Another family of molecules that falls within the definition of active agents is DHEA, derivatives thereof and chemical or metabolic precursors thereof.

DHEA or dehydroepiandrosterone, also known as 3β-hydroxyandrost-5-en-17-one, or dehydroisoandrosterone, but also trans-dehydroandrosterone or prasterone, has the formula:

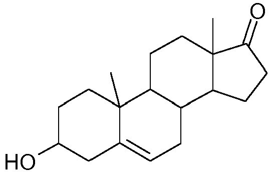

The term "DHEA precursors concerned in the invention" means biological precursors thereof that are capable of transforming DHEA during metabolism, and also chemical precursors thereof that can be transformed into DHEA via an exogenous chemical reaction.

Examples of biological precursors are Δ5-pregnenolone, 17α-hydroxypregnenolone and 17α-hydroxypregnenolone sulfate, without this list being limiting.

The term "chemical precursors of DHEA" especially means saponins and derivatives thereof such as hecogenin (3β,5α,23r)-3-hydroxyspirostan-12-one) and hecogenin acetate, diosgenin (5-spirosten-3β-ol), smilagenin and sarsapogenin, and also natural extracts containing them, in particular fenugreek and Dioscorea plants such as wild yam root, without this list being limiting.

The term "DHEA derivatives" means both the metabolic derivatives thereof and the chemical derivatives thereof. Metabolic derivatives that may especially be mentioned include Δ5-androstene-3,17-diol, and especially 5-androstene-3β,17β-diol, Δ4-androstene-3,17-dione, 7-hydroxy-DHEA (7α-hydroxy-DHEA or 7β-hydroxy-DHEA) and 7-keto-DHEA, which is itself a metabolite of 7β-hydroxy-DHEA, without this list being limiting.

7α-Hydroxy-DHEA is, along with 5-androstene 3β,17β-diol, a major metabolite of DHEA, obtained via the action of 7α-hydroxylase on DHEA. 7β-Hydroxy-DHEA is a minor metabolite of DHEA, obtained via reaction of 7β-hydroxylase on DHEA.

The 7-hydroxy-DHEA preferably used in the compositions according to the present invention is 7α-hydroxy-DHEA. A process for preparing this compound is described in patent applications FR 2 771 105 and WO 94/08588.

As chemical derivatives of DHEA, mention may also be made of DHEA salts and in particular the water-soluble salts such as DHEA sulfate; DHEA esters such as DHEA esters of hydroxycarboxylic acids, in particular those described in patent US 5 736 537, or DHEA salicylate, DHEA acetate, DHEA valerate (or n-heptanoate) and DHEA enanthate.

Mention may also be made of DHEA carbamates, DHEA 2-hydroxymalonate esters and DHEA amino acid esters. Finally, mention may be made of 3β-acetoxy-7-oxo-DHEA, which may especially be prepared as described in patents US-5 869 709 and US-6 111 118. This list is obviously not limiting.

The concentration of DHEA or of derivatives or chemical or metabolic precursors thereof in the composition according to the present invention may advantageously range from 0.001 % to 30% by weight, preferably from 0.01% to 20% by weight and even more preferably from 0.01% to 10% by weight relative to the total weight of the composition. These compounds will be in dissolved form between 20°C and 90°C.

According to another particular form of the invention, the derivative(s) of formula (I) in accordance with the invention are used as sole solvent for the said lipophilic active agent(s).

The compositions in accordance with the invention may comprise, in addition to the active agents and screening agents mentioned previously, other UVA-active and/or UVB-active organic UV-screening agents that are water-soluble or insoluble in the cosmetic solvents commonly used.

Among the UVA-active UV-screening agents that are capable of absorbing UV from 320 to 400 nm, mention may be made of:
- terephthalylidenedicamphorsulfonic acid manufactured under the name Mexoryl SX by Chimex,
- bis-benzazolyl derivatives as described in patents EP 669 323, and US 2 463 264 and more particularly the compound disodium phenyldibenzimidazotetrasulfonate sold under the trade name Neo Heliopan AP by Haarmann and Reimer.

Among the UVB-active UV-screening agents that are capable of absorbing UV from 280 to 320 nm, mention may be made of:
- p-aminobenzoic (PABA) derivatives such as:
   - PABA,
   - Glyceryl PABA, and
   - PEG-25 PABA sold under the name Uvinul P25 by BASF,
- Phenylbenzimidazolesulfonic acid sold in particular under the trade name Eusolex 232 by Merck,
- ferulic acid,
- p-methoxycinnamic acid,
- DEA methoxycinnamate,
- benzylidenecamphorsulfonic acid manufactured under the name Mexoryl SL by Chimex,
- camphorbenzalkonium methosulfate manufactured under the name Mexoryl SO by Chimex.

Among the water-soluble UVA-active and UVB-active UV-screening agents, mention may be made of:
- Benzophenone-4 sold under the trade name Uvinul MS40 by BASF,
- Benzophenone-5, and
- Benzophenone-9.

Among the insoluble UV-screening agents, mention may be made of:
- methylenebis(benzotriazolyl)tetramethylbutylphenol sold in solid form under the trade name Mixxim BB/100 by Fairmount Chemical, or in micronized form as an aqueous dispersion under the trade name Tinosorb M by BASF,
- the symmetrical triazine screening agents described in patent US 6 225 467, patent application WO 2004/085 412 (see compounds 6 and 9) or the document Symmetrical Triazine Derivatives, IP.COM Journal, IP.COM INC West Henrietta, NY, US (20 September 2004), especially 2,4,6-tris(biphenyl)-1,3,5-triazines (in particular 2,4,6-tris(biphenyl-4-yl-1,3,5-triazine) and 2,4,6-tris(terphenyl)-1,3,5-triazine which is also mentioned in Beiersdorf patent applications WO 06/035 000, WO 06/034 982, WO 06/034 991, WO 06/035 007, WO 2006/034 992 and WO 2006/034 985.

Of course, a person skilled in the art will take care to choose the optional additional screening agent or agents and/or their amounts so that the advantageous properties intrinsically attached to the compositions in accordance with the invention are not, or not substantially, detrimentally affected by the envisaged addition or additions.

The compositions according to the invention can also comprise agents for the artificial tanning and/or browning of the skin (self-tanning agents) and more particularly dihydroxyacetone (DHA). They are preferably present in amounts ranging from 0.1% to 10% by weight, relative to the total weight of the composition.

The compositions in accordance with the present invention may also comprise, besides water, standard cosmetic adjuvants chosen especially from fatty substances such as oils, organic solvents, ionic or nonionic, hydrophilic or lipophilic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, acidifying or basifying agents or any other ingredient usually used in cosmetics and/or dermatology.

Mention may be made, as oils, of mineral oils (liquid paraffin); vegetable oils (sweet almond, macadamia, blackcurrant seed or jojoba oil); synthetic oils, such as perhydrosqualene, fatty alcohols, fatty amides (such as isopropyl lauroyl sarcosinate, sold under the name Eldew SL-205 by the company Ajinomoto), fatty acids or esters (such as C12-C15 alkyl benzoates, sold under the trade name Finsolv TN or Witconol TN by the company Witco, 2-ethylphenyl benzoate, such as the commercial product sold under the name X-Tend 226® by the company ISP, octyl palmitate, isopropyl lanolate, triglycerides, including those of capric/caprylic acids, for instance Miglyol® 812 sold by the company Dynamit Nobel, dicaprylyl carbonate, sold under the name Cetiol CC by the company Cognis), or oxyethylenated or oxypropylenated fatty esters and ethers; silicone oils (cyclomethicone, polydimethylsiloxanes or PDMSs); fluorinated oils; or polyalkylenes.

Waxy compounds that may be mentioned include carnauba wax, beeswax, hydrogenated castor oil, polyethylene waxes and polymethylene waxes, for instance the product sold under the name Cirebelle 303 by the company Sasol.

Among the organic solvents that may be mentioned are lower alcohols and polyols. The latter can be chosen from glycols and glycol ethers, such as ethylene glycol, propylene glycol, butylene glycol, dipropylene glycol or diethylene glycol.

Hydrophilic thickeners that may be mentioned include carboxyvinyl polymers such as the Carbopol products (Carbomers) and the Pemulen products (acrylate/C₁₀-C₃₀-alkylacrylate copolymer); polyacrylamides, for instance the crosslinked copolymers sold under the names Sepigel 305 (CTFA name : polyacrylamide/C13-14 isoparaffin/Laureth 7) or Simulgel 600 (CTFA name : acrylamide/sodium acryloyldimethyl taurate copolymer/isohexadecane/polysorbate 80) by the company SEPPIC; 2-acrylamido-2-methylpropanesulfonic acid polymers and copolymers, optionally crosslinked and/or neutralized, for instance poly(2-acrylamido-2-methylpropanesulfonic acid) sold by the company Hoechst under the trade name Hostacerin AMPS (CTFA name: ammonium polyacryloyldimethyl taurate) or Simulgel 800 sold by the company SEPPIC (CTFA name : sodium polyacryloyldimethyl taurate/polysorbate 80/isohexadecane); copolymers of 2-acrylamido-2-methylpropanesulfonic acid and of hydroxyethyl acrylate, for instance Simulgel NS and Sepinov EMT 10 sold by the company SEPPIC; cellulose derivatives such as hydroxyethyl cellulose; polysaccharides and especially gums such as xanthan gum; and mixtures thereof.

Lipophilic thickeners that may be mentioned include synthetic polymers, such as the poly(C10-C30 alkyl acrylates) sold under the names Intelimer IPA 13-1 and Intelimer IPA 13-6 by the company Landec, or modified clays, such as hectorite and its derivatives, for instance the products sold under the name Bentone.

Mention may be made, among active agents, of:
- agents for combating pollution and/or agents for combating free radicals;
- depigmenting agents and/or propigmenting agents;
- antiglycation agents;
- calmatives;
- NO-synthase inhibitors;
- agents for stimulating the synthesis of dermal or epidermal macromolecules and/or for preventing their degradation;
- agents for stimulating fibroblast proliferation;
- agents for stimulating keratinocyte proliferation;
- muscle relaxants;
- tensioning agents;
- matting agents;
- keratolytic agents;
- desquamating agents;
- moisturizers;
- anti-inflammatory agents;
- agents that act on the energy metabolism of cells;
- insect repellents;
- substance P or substance CRGP antagonists;
- hair-loss counteractants and/or hair restorers;
- anti-wrinkle agents.

Needless to say, a person skilled in the art will take care to select the optional additional compound(s) mentioned above and/or the amounts thereof such that the advantageous properties intrinsically associated with the compositions in accordance with the invention are not, or are not substantially, adversely affected by the envisaged addition(s).

The compositions according to the invention may be prepared according to the techniques that are well known to those skilled in the art. They may in particular be in the form of a simple or complex emulsion (O/W, W/O, O/W/O or W/O/W) such as a cream, a milk or a cream-gel; in the form of an aqueous gel; in the form of a lotion. They may optionally be packaged as an aerosol and may be in the form of a mousse or a spray.

The compositions according to the invention are preferably in the form of an oil-in-water or water-in-oil emulsion.

The emulsions generally contain at least one emulsifier chosen from amphoteric, anionic, cationic and nonionic emulsifiers, used alone or as a mixture. The emulsifiers are appropriately chosen according to the emulsion to be obtained (W/O or O/W). The emulsions may also contain stabilizers of other types, for instance fillers, or gelling or thickening polymers.

As emulsifiers that may be used for the preparation of the W/O emulsions, examples that may be mentioned include alkyl esters or ethers of sorbitan, of glycerol or of sugars; silicone surfactants, for instance dimethicone copolyols, such as the mixture of cyclomethicone and of dimethicone copolyol, sold under the name DC 5225 C by the company Dow Corning, and alkyldimethicone copolyols such as laurylmethicone copolyol sold under the name Dow Corning 5200 Formulation Aid by the company Dow Corning; cetyldimethicone copolyol, such as the product sold under the name Abil EM 90R by the company Goldschmidt, and the mixture of cetyldimethicone copolyol, of polyglyceryl isostearate (4 mol) and of hexyl laurate, sold under the name Abil WE O9 by the company Goldschmidt. One or more coemulsifiers may also be added thereto, which may be chosen advantageously from the group comprising polyol alkyl esters.

Polyol alkyl esters that may especially be mentioned include polyethylene glycol esters, for instance PEG-30 dipolyhydroxystearate, such as the product sold under the name Arlacel P135 by the company ICI.

Glycerol and/or sorbitan esters that may be mentioned include, for example, polyglyceryl isostearate, such as the product sold under the name Isolan GI 34 by the company Goldschmidt, sorbitan isostearate, such as the product sold under the name Arlacel 987 by the company ICI, sorbitan glyceryl isostearate, such as the product sold under the name Arlacel 986 by the company ICI, and mixtures thereof.

For the O/W emulsions, examples of emulsifiers that may be mentioned include nonionic emulsifiers such as oxyalkylenated (more particularly polyoxyethylenated) esters of fatty acids and of glycerol; oxyalkylenated esters of fatty acids and of sorbitan; oxyalkylenated (oxyethylenated and/or oxypropylenated) esters of fatty acids, such as the PEG 100 stearate/glyceryl stearate mixture sold, for example, by ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by the company Henkel under the respective names Plantaren 2000 and Plantaren 1200, cetostearyl glucoside, optionally as a mixture with cetostearyl alcohol, sold, for example, under the name Montanov 68 by the company SEPPIC, under the name Tegocare CG90 by the company Goldschmidt and under the name Emulgade KE3302 by the company Henkel, and also arachidyl glucoside, for example in the form of a mixture of arachidyl alcohol, behenyl alcohol and arachidyl glucoside, sold under the name Montanov 202 by the company SEPPIC. According to one particular embodiment of the invention, the mixture of the alkyl polyglucoside as defined above with the corresponding fatty alcohol can be in the form of a self-emulsifying composition, for example as described in document WO-A-92/06778.

Among the other emulsifiers that will be used more particularly are isophthalic acid or sulfoisophthalic acid polymers, and in particular phthalate/sulfoisophthalate/glycol copolymers, for example the diethylene glycol/phthalate/isophthalate/1,4-cyclohexanedimethanol copolymer (INCI name: Polyester-5) sold under the name Eastman AQ Polymer (AQ35S, AQ38S, AQ55S and AQ48 Ultra) by the company Eastman Chemical.

When it is an emulsion, the aqueous phase of this emulsion may comprise a nonionic vesicular dispersion prepared according to known processes (Bangham, Standish and Watkins, J. Mol. Biol. 13, 238 (1965), FR 2 315 991 and FR 2 416 008).

The compositions according to the invention find their application in a large number of cosmetic applications for the skin, the lips, the nails, the eyelashes, the eyebrows and the hair, including the scalp, especially for protecting and/or caring for the skin, the lips and/or the hair, and/or for making up the skin and/or the lips and/or the nails and/or the eyelashes and/or the eyebrows.

Another subject of the present invention consists of the use of the compositions according to the invention as defined above for the manufacture of cosmetic products for treating the skin, the lips, the nails, the hair, the eyelashes, the eyebrows and/or the scalp, especially care products, antisun protection products and makeup products.

The cosmetic compositions according to the invention may be used, for example, as makeup products.

The cosmetic compositions according to the invention may be used, for example, as care products and/or antisun protection products for the face and/or the body. These compositions according to the invention may be of liquid to semi-liquid consistency, such as milks, more or less rich creams, cream-gels or pastes. They may optionally be conditioned in aerosol form and may be in the form of a mousse or a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and also aerosol pumps using compressed air as propellant. These devices are described in patents US 4 077 441 and US 4 850 517.

The compositions conditioned in aerosol form in accordance with the invention generally contain conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

Concrete, but in no way limiting, examples illustrating the invention will now be given.

### COMPARED SOLUBILITIES OF LIPOPHILIC UV-SCREENING AGENTS BETWEEN SOLVENTS OF THE PRIOR ART AND THE SOLVENTS OF THE INVENTION

**Filter 1:** Bis(ethylhexyloxyphenol)methoxyphenyltriazine (Tinosorb S) 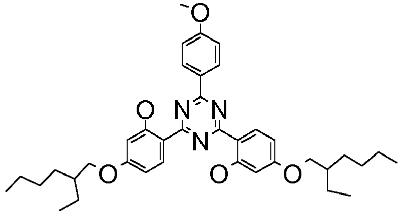
**Filter 2:** Ethylhexyl triazone (Uvinul T150) 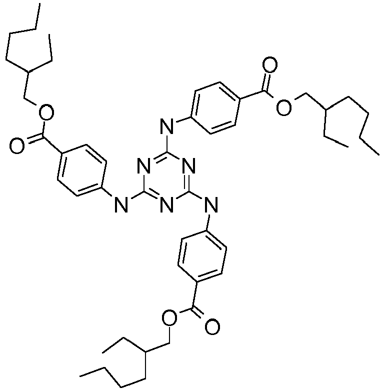
**Filter 3:** Butylmethoxydibenzoylmethane (Parsol 1789) 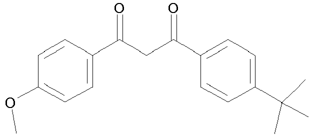
**Filter 4:** Bis(butyl benzoate)diaminotriazine aminopropyltrisiloxane, 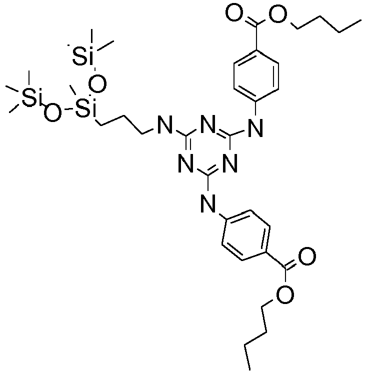
**Filter 5:** Diethylhexylbutamidotriazone (Uvasorb HEB)

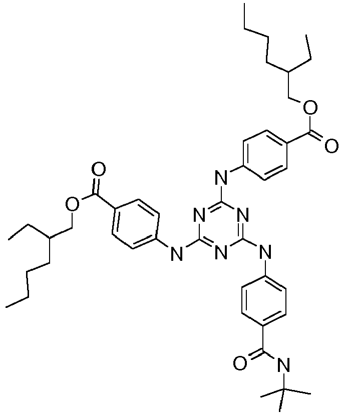

**Comparative oil 1:** Eldew SL-205 from Ajinomoto: Isopropyl N-lauroyl sarcosinate of formula: 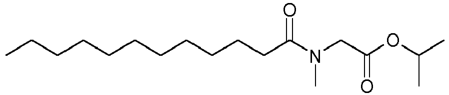
**Comparative oil 2:** Finsolv TN: (C₁₂-C₁₅)alkyl benzoate
**Comparative oil 3:** Miglyol 812: Caprylic-capric acid triglycerides
**Comparative oil 4:** Rhodiasolv: dimethyl-2-methyl glutarate

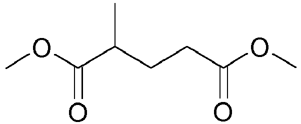

### Procedure:

X mg of product are introduced into Y mg of oil; with gentle heating (less than 60°C) and using a sonicator for 1 minute, the solution obtained is left at the laboratory temperature for 1 month; the state of this solution is observed; if no crystals or oily deposit are visible, the solubility of the product is considered as being greater than X×100/(X+Y) on a weight/weight basis; when crystals or an oily deposit appear, the test is repeated with 5% less of product.

**Table 1**

| **Solvent** | **Filter 1** | **Filter 2** | **Filter 3** | **Filter 4** | **Filter 5** |
|---|---|---|---|---|---|
| **Comparative oil 1** | < 12% | < 15% | < 10% | < 30% | > 30% |
| **Comparative oil 2** | 4% | 5% | 6% | 15% | > 15% |
| **Comparative oil 3** | 4% | 4% | 5% | 14% | > 10% |
| **Comparative oil 4** | < 10% | < 8% | < 15% | < 15% | < 20% |
| **Compound (s) of the invention** | > 30% | > 30% | > 20% | > 30% | > 30% |
| **Compound (w) of the invention** | > 20% | > 20% | > 20% | > 20% | > 30% |

### FORMULATION EXAMPLES 1 to 4

The following formulations were prepared: the amounts are expressed as weight percentages relative to the total weight of the composition.

| **Composition** | **Ex.1** | **Ex. 2** | **Ex. 3** | **Ex. 4** |
|---|---|---|---|---|
| Phase A | | | | |
| Polydimethylsiloxane | 0.5 | 0.5 | 0.5 | 0.5 |
| Preserving agents | 1 | 1 | 1 | 1 |
| Stearic acid | 1.5 | 1.5 | 1.5 | 1.5 |
| Glyceryl monostearate/PEG-100 stearate mixture | 1 | 1 | 1 | 1 |
| Mixture of cetylstearyl glucoside and of cetyl and stearyl alcohols | 2 | 2 | 2 | 2 |
| Cetyl alcohol | 0.5 | 0.5 | 0.5 | 0.5 |
| Compound (s) of the invention | 20 | | 15 | - |
| Compound (w) of the invention | | 20 | | 15 |
| 1-(4-Methoxy-1-benzofur-5-yl)-3-phenylpropane-1,3-dione (Pongamol) | 5 | 5 | | |
| Dineopentyl 2-(3,3-diphenylprop-2-enylidene)malonate | - | - | 5 | 5 |

| Phase B | | | | |
|---|---|---|---|---|
| Deionized water | qs 100 | qs 100 | qs 100 | qs 100 |
| Glycerol | 5 | 5 | 5 | 5 |
| Xanthan gum | 0.2 | 0.2 | 0.2 | 0.2 |
| Monocetyl phosphate | 1 | 1 | 1 | 1 |

| Phase C | | | | |
|---|---|---|---|---|
| Isohexadecane | 1 | 1 | 1 | 1 |
| Acrylic acid/stearyl methacrylate copolymer | 0.2 | 0.2 | 0.2 | 0.2 |
| Triethanolamine | qs pH | qs pH | qs pH | qs pH |

### Procedure:

The aqueous phase (Phase B) comprising all of its ingredients is heated to 80°C in a water bath. The fatty phase (Phase A) comprising all of its ingredients is heated to 80°C in a water bath. A is emulsified in B with stirring of rotor-stator type (device from Moritz). Phase C is incorporated and the mixture is allowed to return to ambient temperature with moderate stirring. The triethanolamine is introduced so as to adjust the pH to the value desired at the end of manufacture. The antisun emulsions obtained are stable on storage and do not show any crystals or precipitate.

## Claims

1. Use of at least one 2-methylsuccinic acid diester derivative of formula (I) below and/or an optical isomer thereof and/or solvate thereof: 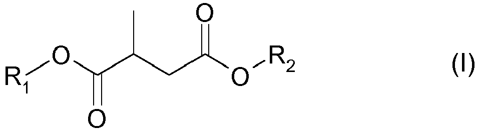 in which:
R₁ and R₂, which may be identical or different, denote a linear or branched C₁-C₁₂ alkyl radical, R₁ and R₂ not simultaneously denoting a methyl radical,
in a composition comprising, in a cosmetically acceptable medium, at least one liquid fatty phase and at least one lipophilic active agent selected from lipophilic organic UV-screening agents, for dissolving the said active agent in the said liquid fatty phase and/or for improving the solubility of the said active agent in the said fatty phase.

2. Use according to Claim 1, in which the compound of formula (I) is chosen from compounds (a) to (ab) below:

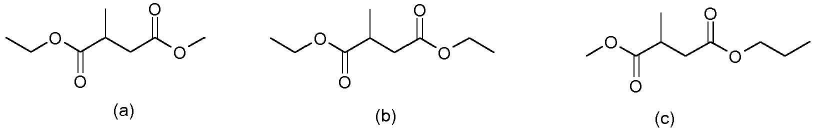

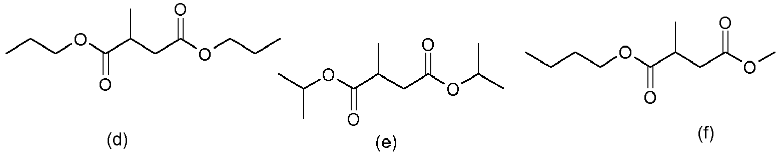

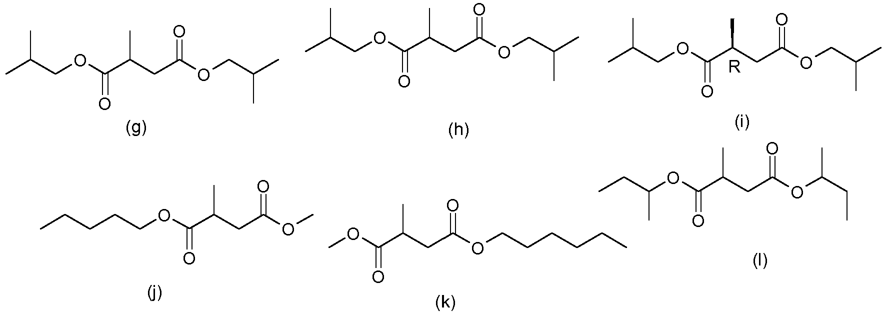

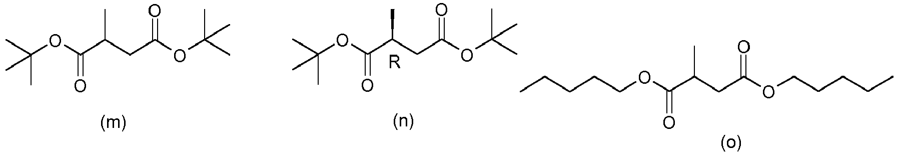

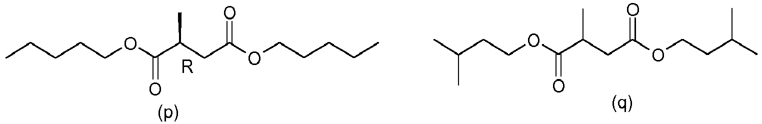

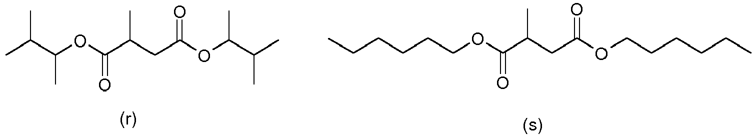

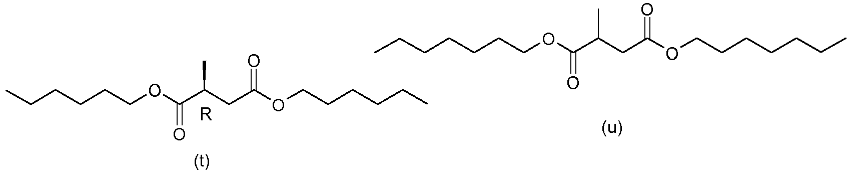

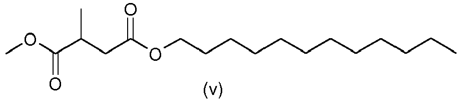

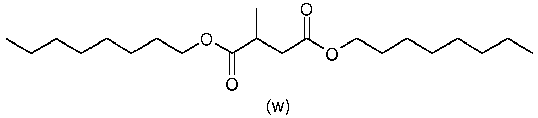

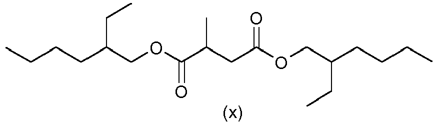

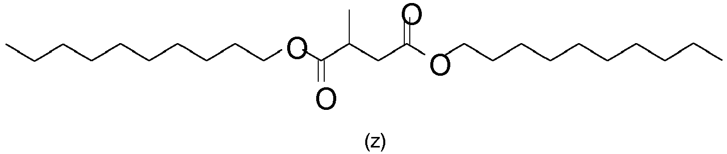

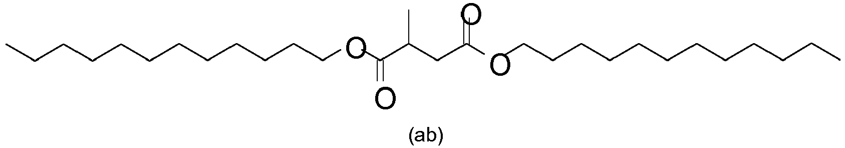

3. Use according to any one of Claims 1 to 2, in which the compound of formula (I) is chosen from compounds (g), (j), (k), (I), (o), (q), (s), (u), (v), (w), (x), (z) and (ab) and even more particularly compounds (s), (w) and (x).

4. Use according to any one of Claims 1 to 3, in which the compound(s) of formula (I) in accordance with the invention constitute(s) the sole solvent for the lipophilic active agent(s).

5. Use according to any one of Claims 1 to **4,** in which the lipophilic organic UV-screening agent is chosen from para-aminobenzoic acid derivatives, salicylic derivatives, cinnamic derivatives, benzophenone and aminobenzophenone derivatives, anthranilic derivatives, dibenzoylmethane derivatives, β,β-diphenylacrylate derivatives, benzylidenecamphor derivatives, benzotriazole derivatives, triazine derivatives, bis-resorcinyl triazine derivatives, imidazoline derivatives, benzalmalonate derivatives, 4,4-diarylbutadiene derivatives, benzoxazole derivatives, merocyanin derivatives, diphenyl butadiene malonate or malononitrile derivatives, chalcone derivatives and merocyanin derivatives, and mixtures thereof.

6. Use according to Claim 5, in which the lipophilic organic UV-screening agent is chosen from dibenzoylmethane derivatives, triazine derivatives, bis-resorcinyl triazine derivatives, benzotriazole derivatives, chalcone derivatives, benzophenone or aminobenzophenone derivatives, and derivatives of the diphenylbutadiene malonate or malononitrile family, or mixtures thereof.

7. Composition comprising, in a cosmetically acceptable medium, at least one liquid fatty phase, **characterized in that** it contains at least one 2-methylsuccinic acid diester derivative of formula (I) and/or an optical isomer and/or solvate thereof as defined in any one of Claims 1 to 3 and at least one lipophilic active agent selected from lipophilic organic UV-screening agents.

8. Composition according to Claim 7, in which the lipophilic active agent is chosen from lipophilic organic UV-screening agents as defined in either of Claims 5 and 6

9. Compounds of formulae (x), (z) and (ab).

## Patentansprüche

1. Verwendung mindestens eines 2-Methylbernsteinsäurediesterderivats der nachstehenden Formel (I) und/oder eines optischen Isomers davon und/oder Solvats davon: 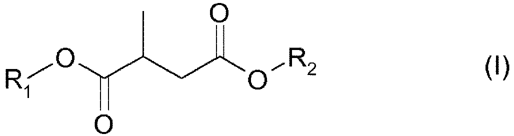 wobei:
R₁ und R₂, die gleich oder verschieden sein können, für einen linearen oder verzweigten C₁-C₁₂-Alkylrest stehen, wobei R₁ und R₂ nicht gleichzeitig für einen Methylrest stehen,
in einer Zusammensetzung, die in einem kosmetisch unbedenklichen Medium mindestens eine flüssige Fettphase und mindestens einen lipophilen Wirkstoff, der aus lipophilen organischen UV-Filtersubstanzen ausgewählt ist, umfasst, zum Lösen des Wirkstoffs in der flüssigen Fettphase und/oder zur Verbesserung der Löslichkeit des Wirkstoffs in der Fettphase.

2. Verwendung nach Anspruch 1, wobei die Verbindung der Formel (I) aus den nachstehenden Verbindungen (a) bis (ab) ausgewählt ist:

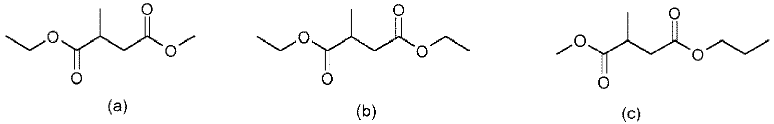

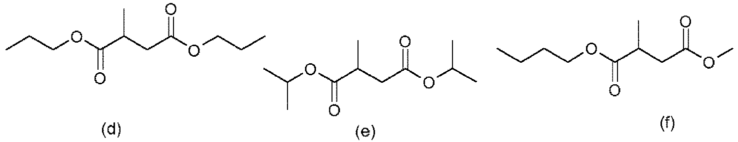

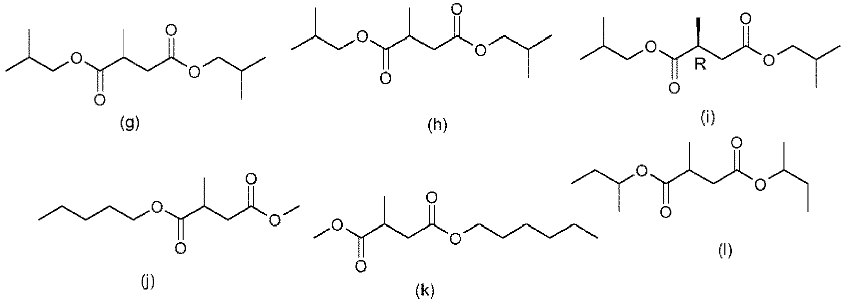

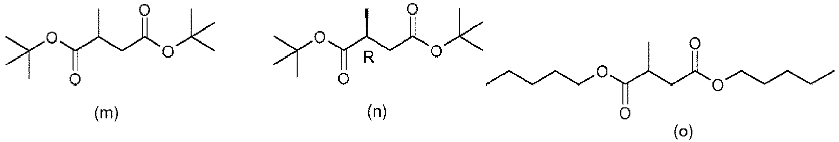

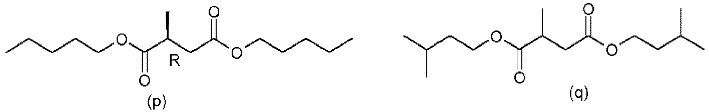

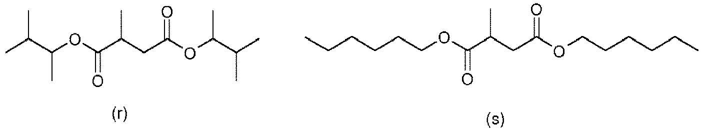

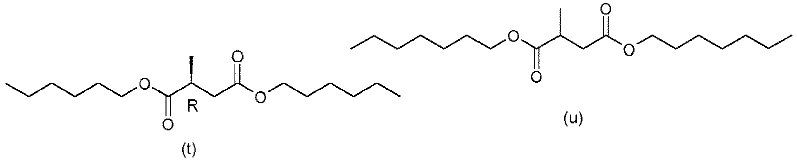

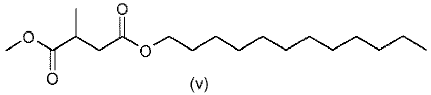

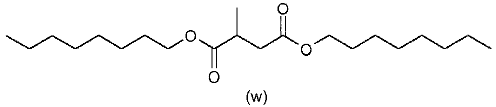

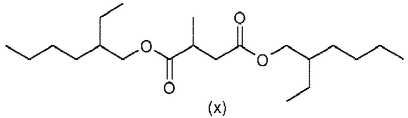

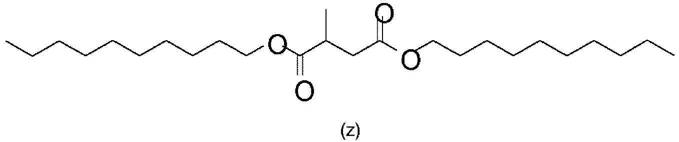

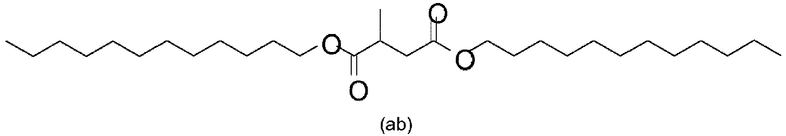

3. Verwendung nach einem der Ansprüche 1 bis 2, wobei die Verbindung der Formel (I) aus den Verbindungen (g), (j), (k), (1), (o), (q), (s), (u), (v), (w), (x), (z) und (ab) und noch spezieller den Verbindungen (s), (w) und (x) ausgewählt ist.

4. Verwendung nach einem der Ansprüche 1 bis 3, wobei die Verbindung bzw. Verbindungen der Formel (I) gemäß der Erfindung das einzige Lösungsmittel für den lipophilen Wirkstoff bzw. die lipophilen Wirkstoffe darstellt bzw. darstellen.

5. Verwendung nach einem der Ansprüche 1 bis 4, wobei die lipophile organische UV-Filtersubstanz aus para-Aminobenzoesäurederivaten, Salicylsäurederivaten, Zimtsäurederivaten, Benzophenon- und Aminobenzophenonderivaten, Anthranilsäurederivaten, Dibenzoylmethanderivaten, β,β-Diphenyl-acrylatderivaten, Benzylidencampherderivaten, Benzotriazolderivaten, Triazinderivaten, Bisresorcinyltriazinderivaten, Imidazolinderivaten, Benzalmalonatderivaten, 4,4-Diarylbutadienderivaten, Benzoxazolderivaten, Merocyaninderivaten, Diphenylbutadienmalonat- oder -malononitrilderivaten, Chalkonderivaten und Merocyaninderivaten und Mischungen davon ausgewählt ist.

6. Verwendung nach Anspruch 5, wobei die lipophile organische UV-Filtersubstanz aus Dibenzoylmethanderivaten, Triazinderivaten, Bisresorcinyltriazinderivaten, Benzotriazolderivaten, Chalkonderivaten, Benzophenon- oder Aminobenzophenonderivaten, und Derivaten der Diphenylbutadienmalonat- oder -malononitril-Familie oder Mischungen davon ausgewählt sind.

7. Zusammensetzung, umfassend in einem kosmetisch unbedenklichen Medium mindestens eine flüssige Fettphase, **dadurch gekennzeichnet, dass** sie mindestens ein 2-Methylbernsteinsäurediesterderivat der Formel (I) und/oder ein optisches Isomer und/oder Solvat davon gemäß einem der Ansprüche 1 bis 3 und mindestens einen lipophilen Wirkstoff, der aus lipophilen organischen UV-Filtersubstanzen ausgewählt ist, umfasst.

8. Zusammensetzung nach Anspruch 7, wobei der lipophile Wirkstoff aus lipophilen organischen UV-Filtersubstanzen gemäß einem der Ansprüche 5 und 6 ausgewählt ist.

9. Verbindungen der Formel (x), (z) und (ab).

## Revendications

1. Utilisation d'au moins un diester de l'acide 2-méthylsuccinique de formule (I) ci-dessous et/ou d'un isomère optique correspondant et/ou d'un solvate correspondant : 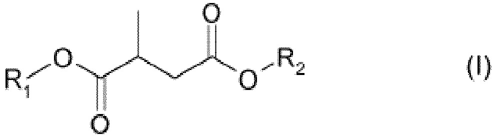 dans laquelle :
R₁ et R₂, qui peuvent être identiques ou différents, désignent un radical C₁₋₁₂-alkyle linéaire ou ramifié, R₁ et R₂ ne désignant pas simultanément un radical méthyle,
dans une composition comprenant, dans un milieu acceptable sur le plan cosmétique, au moins une phase grasse liquide et au moins un agent actif lipophile choisi parmi des agents anti-UV organiques lipophiles, pour la dissolution dudit agent actif dans ladite phase grasse liquide et/ou pour l'amélioration de la solubilité dudit agent actif dans ladite phase grasse.

2. Utilisation selon la revendication 1, dans laquelle le composé de formule (I) est choisi parmi les composés (a) à (ab) ci-dessous :

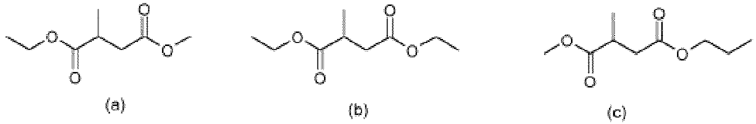

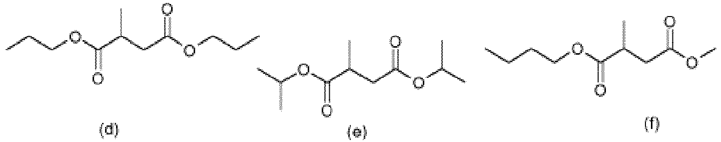

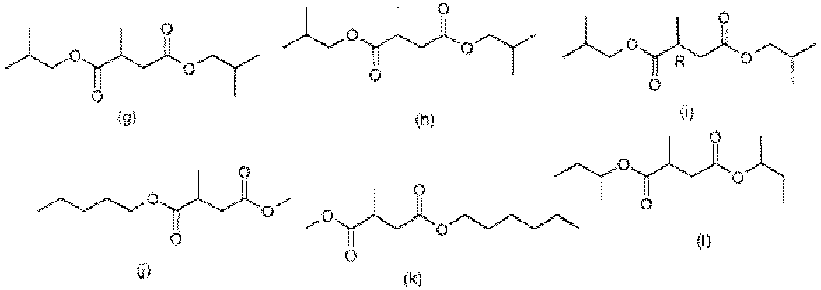

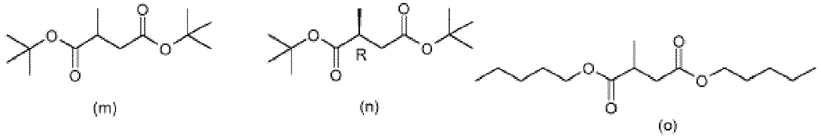

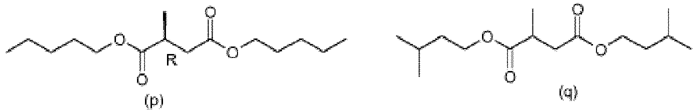

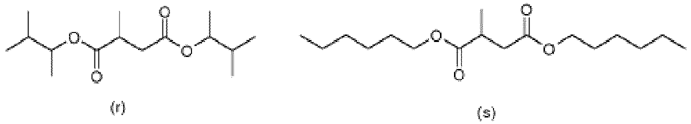

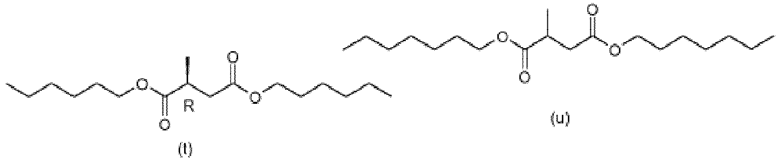

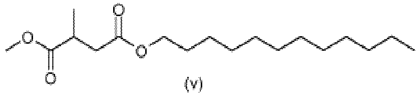

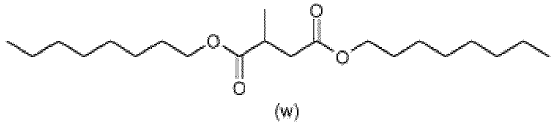

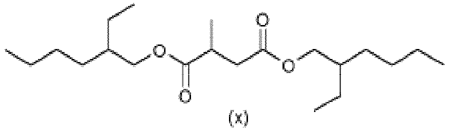

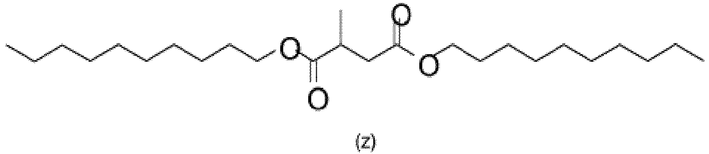

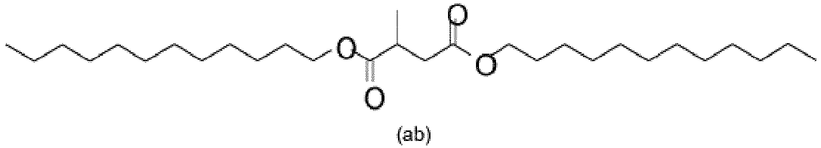

3. Utilisation selon l'une quelconque des revendications 1 à 2, dans laquelle le composé de formule (I) est choisi parmi les composés (g), (j), (k), (l), (o), (q), (s), (u), (v), (w), (x), (z) et (ab) et encore plus particulièrement parmi les composés (s), (w) et (x).

4. Utilisation selon l'une quelconque des revendications 1 à 3, dans laquelle le(s) composé(s) de formule (I) selon l'invention constitue(nt) le seul solvant pour le ou les agent(s) actif(s) lipophile(s).

5. Utilisation selon l'une quelconque des revendications 1 à 4, dans laquelle l'agent anti-UV organique lipophile est choisi parmi des dérivés de l'acide para-aminobenzoïque, des dérivés salicyliques, des dérivés cinnamiques, des dérivés de la benzophénone et de l'aminobenzophénone, des dérivés anthraniliques, des dérivés du dibenzoylméthane, des dérivés de β,β-diphénylacrylate, des dérivés de benzylidènecamphre, des dérivés de benzotriazole, des dérivés de triazine, des dérivés de bis-résorcinyltriazine, des dérivés d'imidazoline, des dérivés de benzalmalonate, des dérivés de 4,4-diarylbutadiène, des dérivés de benzoxazole, des dérivés de mérocyanine, des dérivés du diphénylbutadiène malonate ou malonitrile, des dérivés de chalcone et des dérivés de mérocyanine, et des mélanges correspondants.

6. Utilisation selon la revendication 5, dans laquelle l'agent anti-UV organique lipophile est choisi parmi des dérivés du dibenzoylméthane, des dérivés de triazine, des dérivés de bis-résorcinyltriazine, des dérivés de benzotriazole, des dérivés de chalcone, des dérivés de la benzophénone et de l'aminobenzophénone, et des dérivés de la famille du diphénylbutadiène malonate ou malonitrile, ou des mélanges correspondants.

7. Composition comprenant, dans un milieu acceptable sur le plan cosmétique, au moins une phase grasse liquide, **caractérisée en ce qu'**elle contient au moins un dérivé diester de l'acide 2-méthylsuccinique de formule (I) et/ou un isomère optique et/ou un solvate correspondant tel que défini selon l'une quelconque des revendications 1 à 3 et au moins un agent actif lipophile choisi parmi des agents anti-UV organiques lipophiles.

8. Composition selon la revendication 7, dans laquelle l'agent actif lipophile est choisi parmi des agents anti-UV organiques lipophiles tels que définis dans l'une des revendications 5 et 6.

9. Composés de formule (x), (z) et (ab).
